(19) Europäisches Patentamt
European Patent Office
Office européen des brevets

(11) Veröffentlichungsnummer: **0 259 265 B1**

# (12) EUROPÄISCHE PATENTSCHRIFT

(45) Veröffentlichungstag der Patentschrift: **11.09.91**

(51) Int. Cl.5: **C07D 209/48**, A01N 43/38, C07C 321/24, C07C 215/74

(21) Anmeldenummer: **87810491.8**

(22) Anmeldetag: **28.08.87**

Die Akte enthält technische Angaben, die nach dem Eingang der Anmeldung eingereicht wurden und die nicht in dieser Patentschrift enthalten sind.

## (54) 3-Methylphthalimide.

(30) Priorität: **03.09.86 CH 3538/86**

(43) Veröffentlichungstag der Anmeldung:
**09.03.88 Patentblatt 88/10**

(45) Bekanntmachung des Hinweises auf die Patenterteilung:
**11.09.91 Patentblatt 91/37**

(84) Benannte Vertragsstaaten:
**AT BE CH DE ES FR GB GR IT LI LU NL SE**

(56) Entgegenhaltungen:
**EP-A- 0 061 741**
**CH-A- 646 424**
**DE-B- 1 518 404**
**US-A- 4 292 070**

**PATENT ABSTRACTS OF JAPAN, unexamined applications, Field C, Band 9, Nr. 218, 5. September 1985 THE PATENT OFFICE JAPANESE GOVERNMENT Seite 20 C 301**

(73) Patentinhaber: **CIBA-GEIGY AG**
**Klybeckstrasse 141**
**CH-4002 Basel(CH)**

(72) Erfinder: **Böhner, Beat, Dr.**
**Hügelweg 3**
**CH-4102 Binningen(CH)**
Erfinder: **Moser, Hans, Dr.**
**Maispracherstrasse 12**
**CH-4312 Magden(CH)**
Erfinder: **Pissiotas, Georg, Dr.**
**Am Sonnenrain 71**
**W-7850 Lörrach(DE)**

**EP 0 259 265 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

CHEMICAL ABSTRACTS, Band 101, Nr. 17, 22. Oktober 1984, Columbus, Ohio, USA; SUMITOMO CHEMICAL CO., LTD.: "N-phenyltetrahydrophtalamic acid derivatives" Seite 685, Spalte 1, Zusammenfassung Nr. 151 490q

CHEMICAL ABSTRACTS, Band 103, Nr. 7, 19. August 1985, Columbus, Ohio, USA; LANGLOIS, BERNARD et al.: "m-Substituted anilines" Seite 624, Spalte 2, Zusammenfassung Nr. 71 041v

CHEMICAL ABSTRACTS, Band 70, Nr. 23, 9. Juni 1969, Columbus, Ohio, USA; RUYLE, WILLIAM V. et al.: "Phenylbenzoic acid" Seite 306, Spalte 2, Zusammenfassung Nr. 106 209k

CHEMICAL ABSTRACTS, Band 102, Nr. 5, 4. Februar 1985, Columbus, Ohio, USA; NAGANO, EIKI et al.: "Pyridotriazolium compounds, and their use" Seite 578, Spalte 1, Zusammenfassung Nr. 45 951b

CHEMICAL ABSTRACTS, Band 85, Nr. 25, 20. Dezember 1976, Columbus, Ohio, USA; NAJER, HENRY et al.: "Monosubstituted piperazine derivative" Seite 560, Spalte 2, Zusammenfassung Nr. 192 769g

CHEMICAL ABSTRACTS, Band 103, Nr. 11, 16. September 1985, Columbus, Ohio, USA; SUMITOMO CHEMICAL CO., LTD.: "5-Amino-4-fluoro-2-halophenyl-sulfide" Seite 586, Spalte 1, Zusammenfassung Nr. 87 630x

## Beschreibung

Die vorliegende Erfindung betrifft neue Derivate des N-Phenyltetrahydrophthalimids mit herbizider und pflanzenwuchsregulierender Wirkung, agrochemische Mittel, welche diese Substanzen als Wirkstoffe enthalten, die Verwendung der neuen N-Phenyltetrahydrophthalimide zur selektiven Bekämpfung von Unkräutern oder zur Regulierung des Pflanzenwuchses sowie Verfahren zur Herstellung dieser neuen Verbindungen. Ferner betrifft die Erfindung auch neue Zwischenprodukte, welche zur Herstellung der neuen Wirkstoffe dienen.

Aus der EP-A 61741 sind herbizid wirksame N-Phenyltetrahydrophthalsäureimide bekannt geworden. Diese unter anderem im Phenylimidteil 2,4-dihalogen-5-alkoxy substituierten Verbindungen weisen jedoch im 3,4,5,6-Tetra-hydrophthalsäurerest keinen Methylsubstituenten auf. Als Einzelverbindung sei das N[4-Chlor-2-fluor-5-propoxy-phenyl]-3,4,5,6-tetrahydrophthalsäureimid genannt.

Des weiteren sind aus der japanischen Offenlegungsschrift JP 75,142,730 im Phenylimidteil in den Positionen 4 und 5 mono- bzw. disubstituierte 3-Alkyltetrahydrophthalimide - wie etwa das N-(4-Chlorphenyl)-3-methyl-3,4,5,6-tetrahydrophthalsäureimid - bekannt geworden.

Die aus dem Stand der Technik bekannten Phthalimide, zeigen bei an sich guter herbizider Wirkung geringe Selektivität.

Es wurde nun überraschenderweise gefunden, dass Verbindungen der Formel I bei guter Selektivität und Kulturpflanzenverträglichkeit herbizid hoch wirksam sind.

Die neuen N-Phenyltetrahydrophthalimid-Derivate entsprechen der Formel I

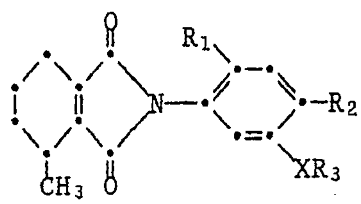

(I)

worin

- $R_1$ Wasserstoff; oder Fluor;
- $R_2$ Halogen;
- X O; oder S;
- $R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxy-arbonyl;

bedeutet.

Die 3-Methyl-tetrahydrophthalimide der Formel I sind an dem die Methylgruppe tragenden Ringkohlenstoffatom $C_3$ unsymmetrisch substituiert und können somit sowohl in der R als auch in der S-Konfiguration (Formel R-I bzw. S-I) vorliegen.

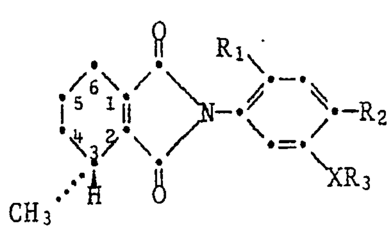

R-(I)

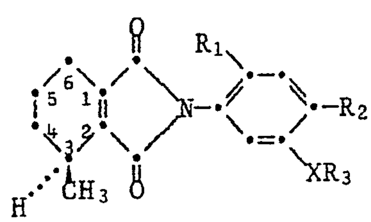

S-(I)

Die Erfindung umfasst beide enantiomeren Formen sowie beliebige Mischungen derselben und das Racemat.

$C_3$-$C_{10}$-Alkenyl sind die einfach, bzw. ab $C_4$ auch mehrfach ungesättigten, cis-trans- wie auch konstitutionsisomeren Radikale; wie etwa das Allyl-, das But-2-en-1-yl- oder das 2-Methyl-prop-2-enyl-radikal.

$C_5$-$C_6$-Cycloalkenyl sind Cyclopentenyl, Cyclohexenyl.

$C_5$-$C_6$-Cycloalkyl sind Cyclopentyl und Cyclohexyl.

$C_3$-$C_{10}$-Alkinyl sind die einfach, bzw. ab $C_4$ auch mehrfach ungesättigten Radikale; wie etwa das Propargyl- oder das But-2-inyl-radikal.

$C_1$-$C_6$-Alkyl ist Methyl, Ethyl, Propyl, iso-Propyl, n-Butyl, iso-Buty, tert-Butyl, sek-Butyl, sowie die isomeren Pentyle, wie n-Pentyl, i-Pentyl (1-Ethyl-propyl), t-Pentyl (1,1-Dimethylpropyl) und die isomeren Hexyl-, radikale.

Halogen ist Fluor, Chlor, Brom und Jod, vorzugsweise Fluor, Chlor und Brom.

In den weiteren Substituenten, welche sich durch die Kombination einzelner Teilelemente ergeben, haben die Teilelemente die im Rahmen der Definitionsbreite gewählte Bedeutung und können durch Kombination von Einzelelementen aus vorstehender Aufzählung frei gewählt werden. Diese Aufzählung bedeutet auch in diesen Fällen keine Einschränkung der Erfindung; sie haben lediglich illustrierenden Charakter.

Bevorzugt sind Verbindungen der Formel I,
worin

- $R_1$ Wasserstoff; oder Fluor;
- $R_2$ Fluor; Chlor; oder Brom;
- X O; oder S;
- $R_3$ $C_3$-$C_5$-Alkenyl; $C_3$-$C_5$-Alkinyl; Cyclopentenyl; Cyclohexenyl; $C_5$-$C_6$-Cycloalkyl; oder $C_1$-$C_6$-Alkyl welches gewünschtenfalls einfach substituiert ist durch $C_1$-$C_4$-Alkoxy-carbonyl,

bedeutet.

Unter den als bevorzugt herausgestellten Verbindungen der Formel I sind als einzelne Gruppen zu nennen:

Gruppe A

Verbindungen der Formel I, worin $R_3$ Methyl, Ethyl, Isopropyl, n-Propyl, n-Butyl, sek-Butyl, iso-Butyl, tert.-Butyl, n-Pentyl, iso-Pentyl oder tert.-Pentyl bedeutet.

Gruppe B

Verbindungen der Formel I, worin $R_3$ Allyl, 2-Methylpropenyl oder But-2-en-1-yl bedeutet.

Gruppe C

Verbindungen der Formel I, worin $R_3$ Propargyl oder But-2-inyl bedeutet.

Gruppe D

Verbindungen der Formel I, worin $R_3$ Cyclopentyl, Cyclohexyl, Cyclopentenyl oder Cyclohexenyl bedeutet.

Gruppe E

Verbindungen der Formel I, worin $R_3$ $C_1$-$C_3$-Alkoxycarbonylmethyl oder $C_1$-$C_3$-Alkoxycarbonylethyl bedeutet.

Gruppe F

Verbindungen der Formel I, worin X Sauerstoff bedeutet.

Gruppe G

Verbindungen der Formel I, worin X Schwefel bedeutet.

Gruppe H

Verbindungen der Formel I, worin $R_1$ Wasserstoff und $R_2$ Chlor oder Brom bedeutet.

Gruppe I

Verbindungen der Formel I, worin $R_1$ Fluor und $R_2$ Chlor oder Brom bedeutet.

Als besonders bevorzugte Einzelverbindungen sind zu nennen:

N-(4-Chlor-2-fluor-5-isopropyloxy-phenyl)-3-methyl-3,4,5,6-tetrahydrophthalsäureimid,
N-(4-Chlor-2-fluor-5-methoxy-phenyl)-3-methyl-3,4,5,6-tetrahydrophthalsäureimid,
N-(4-Chlor-5-ethoxy-2-fluor-phenyl)-3-methyl-3,4,5,6-tetrahydrophthalsäureimid,
N-(5-n-Butyl-4-chlor-2-fluor-phenyl)-3-methyl-3,4,5,6-tetrahydrophthalsäureimid,
N-[4-Chlor-4-fluor-5-(2-methylpropyl)-phenyl]-3-methyl-3,4,5,6-tetrahydrophthalsäureimid und
N-(4-Chlor-5-ethoxycarbonylmethyl-2-fluor-phenyl)-3-methyl-3,4,5,6-tetrahydrophthalsäureimid.

Die Verbindungen der Formel I werden erfindungsgemäss hergestellt,

a) indem man 3-Methyl-3,4,5,6-tetrahydrophthalsäureanhydrid II mit einem Anilin der Formel III,

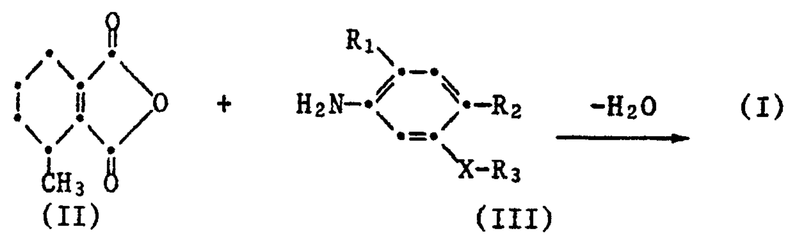

(II) (III)

worin $R_1$, $R_2$, $R_3$ und X die unter Formel I gegebene Bedeutung haben, kondensiert oder

b) in dem man ein Phenol oder Thiophenol der Formel IV mit einer Verbindung der Formel V umsetzt,

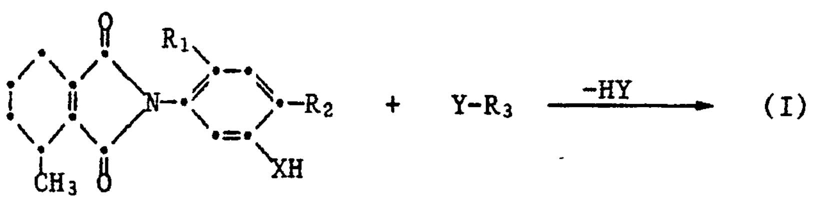

(IV) (V)

worin $R_1$, $R_2$, $R_3$ und X die unter Formel I gegebene Bedeutung haben und Y für eine unter den Reaktionsbedingungen austauschbare Gruppe, wie Halogen, vorzugsweise Chlor, Brom oder Jod oder für einen gewünschtenfalls im Phenylkern alkylierten oder halogenierten Phenylsulfonylrest oder für ein Radikal der Formel $R_3OSO_2$-O steht.

Mit Vorteil führt man die obigen Kondensationsreaktionen in einem inerten organischen Lösungsmittel aus. Die Reaktionstemperatur liegt im allgemeinen zwischen der Raumtemperatur und der Siedetemperatur des Reaktionsgemisches, vorzugsweise wird die Reaktionsmischung zum Rückfluss erhitzt. Die Kondensationsreaktion a) kann durch Zusatz von Kondensationskatalysatoren und Entfernung des entstehenden Reaktionsproduktes Wasser beschleunigt werden. Eine gleiche Wirkung erzielt man durch Zusatz von wasserentziehenden Mitteln, wie beispielsweise Schwefelsäure.

Als Katalysatoren kommen insbesondere dann in Betracht, wenn ein aprotisches Lösungsmittel verwendet wird: p-Toluolsulfonsäure, Benzoesäure, 4-Dimethylaminopyridin, Schwefelsäure, Chlorwasserstoff oder Naphthalinsulfonsäure. Reaktionsführungen gemäss dem oben genannten Typ werden bei der Darstellung von Carbonsäure-Derivaten üblicherweise angewendet. Sie entsprechen der allgemein üblichen Laboratoriumspraxis.

Im Fall der Reaktion b) ist es vorteilhaft unter Basenzusatz zu arbeiten. Geeignete Basen sind u.a. Natrium-, Kalium- und Calciumhydroxid, Alkali- und Erdalkalicarbonate, Amine, wie etwa Triethylamin oder Heterocyclen, wie Pyridin, DABCO etc. Die Reaktion kann auch vorteilhaft unter Phasentransfer-Bedingungen in Zweiphasensystemen durchgeführt werden. Derartige Reaktionen sind dem Fachmann geläufig (z.B. beschrieben in Dehmlow und Dehmlow, Phase Transfer Catalysis; Verlag Chemie; Weinheim 1983).

Als Lösungsmittel eignen sich insbesondere höhersiedende Kohlenwasserstoffe, niedere Alkancarbonsäuren sowie deren Ester und Amide, höhersiedende Ketone und Ether. Beispiele dafür sind Benzol, Toluol, Xylol, Dimethylformamid, Dimethylacetamid, Essigsäure, Ethylacetat, Diisopropylether, Ethylenglykoldimethylether, Tetrahydrofuran, Dioxan, 2-Butanon oder Methylpropylketon.

Das als Ausgangsverbindung verwendete 3-Methyltetrahydrophthalsäureanhydrid II ist literaturbekannt (Beilstein Handbuch der Organ. Chemie Bd. 17[II] 458). In besonders vorteilhafter Weise kann diese Verbindung durch säurekatalysierte Isomerisierung von 3-Methyl-1,2,3,6-tetrahydrophthalsäureanhydrid nach einem literaturbekannten Verfahren (Chem. Abs. 78 71599 k) hergestellt werden.

Die -teilweise- neuen Aniline der Formel III sind ebenfalls Gegenstand der Erfindung. Sie können nach an sich bekannten Verfahren hergestellt werden durch:

a) Reduktion von Nitroverbindungen der Formel VI

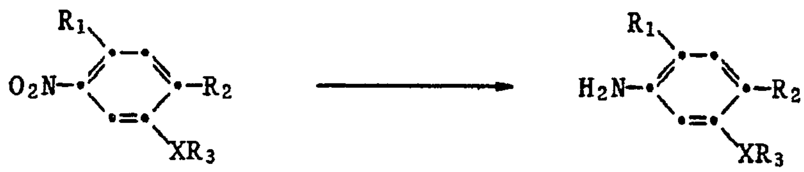

(VI) (III)

z.B. durch katalytische Verfahren (wie $H_2/Pd/C$, $H_2/Pt$ etc.) oder

b) Umsetzung eines Phenols oder Thiophenols der Formel VII mit einer Verbindung der Formel V

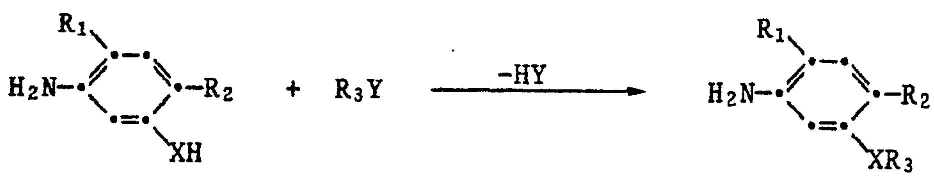


(VII) (V) (III)

gewünschtenfalls unter Basenzusatz.

In den Formeln (III), (V), (VI) und (VII) haben die Reste $R_1$, $R_2$, $R_3$ und X die für Formel I angegebene Bedeutung. Y steht für eine unter den Reaktionsbedingungen austauschbare Gruppe, wie Halogen, vorzugsweise Chlor, Brom oder Jod oder für einen gewünschtenfalls im Phenylkern alkylierten oder halogenierten Phenylsulfonylrest oder für ein Radikal der Formel $R_3$-O-$SO_2$-O.

Desweiteren betrifft die Erfindung die neuen Verbindungen der Formel IV

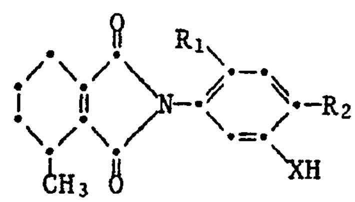

(IV),

worin

- $R_1$ Wasserstoff; oder Fluor;
- $R_2$ Halogen;
- X O; oder S;

bedeutet.

Besonders bevorzugt sind die Verbindungen der Formel IV, in der $R_2$ Fluor, Chlor oder Brom bedeutet und in der $R_1$ und X wie zuvor definiert sind.

Die Verbindungen der Formel IV können analog zu dem bei der Herstellung der Phthalimide I beschriebenen Verfahren erhalten werden durch Umsetzung von 3-Methyl-3,4,5,6-tetrahydrophthalsäureanhydrid II mit einem Anilin der Formel VII

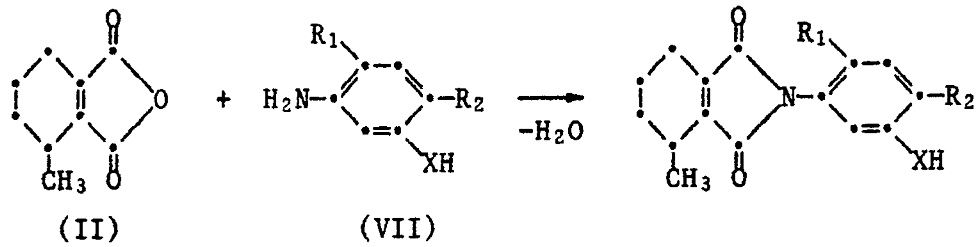

worin $R_1$ Wasserstoff oder Fluor, $R_2$ Halogen und X O oder S bedeutet.

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektivhebizide Eigenschaft gegen Unkräuter aus. Kulturpflanzen wie Roggen, Gerste, Hafer, Weizen, Mais, Hirse, Reis, Baumwolle und Soja bleiben bei niedrigen Aufwandmengen praktische ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Foreml I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Steckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalzezu erwähnen.

Häufiger werden jedoch sogenannte synthetische Tenside verwendet, insbesondere Fettsulfonate,

Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weiter geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxideaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"1986 International Mc. Cutcheon's Emulsifiers & Detergents", Glen Rock, N.J. USA;
H.Stache, "Tensid-Taschenbuch", 2. Auflage., C. Hanser Verlag, München, Wien, 1981;
M. And J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssige Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %

oberflächenaktives Mittel: 0,5 bis 20 %, vorzugsweise 1 bis 15 %
festes Trägermittel: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Während als Handelsware eher konzentrierte Mittel bevorzugt werden, verwendet der Endverbraucher in der Regel verdünnte Mittel. Die Anwendungsform können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

H.1.1. N-(4-Chlor-2-fluor-5-isopropyloxy-phenyl)-3-methyl-3,4,5,6-tetrahydrophthalsäureimid

15,4 g (0,093 mol) 3-Methyl-3,4,5,6-tetrahydrophthalsäureanhydrid werden in 90 ml Toluol gelöst. Nach Zugabe von 18,4 (0,093 mol) 4-Chlor-2-fluor-5-isopropoxyanilin sowie 0,6 g Dimethylaminopyridin heizt man auf, bis das Lösungsmittel und das entstandene Wasser abdestillieren. Nach ca. 2 Std. bei 145 °C Badtemperatur ist die Reaktion beendet. Der Rückstand wird mit Hexan/Essigester 9:1 über eine Kieselgelsäule chromatographiert. Als Hauptfraktion erhält man 26,8 g (82,0 %) der Titelverbindung (Verbindung No. 1.030) der Formel

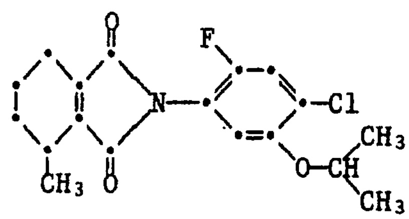

als Oel mit einem $n_D^{22}$ = 1,5458.

H 1.2. N-(4-Chlor-2-fluor-5-isopropyloxy-phenyl)-3-methyl-3,4,5,6-tetrahydrophthalsäureimid

6,1 g (0,02 mol) N-(4-Chlor-2-fluor-5-hydroxyphenyl)-3-methyl-3,4,5,6-tetrahydrophthalsäureimid werden zusammen mit 3,0 g (0,022 mol) Kaliumcarbonat in 50 ml Mehylpropylketon auf 80 °C geheizt. Nach 30 Min. fällt dickes Salz aus. Bei 35 °C gibt man 1,5 ml (0,024 mol) Methljodid zu und lässt das nun wieder dünnflüssige Reaktionsgemisch noch für 2 Std. bei 45 °C ausrühren. Nach dem Abfiltrieren der Salze dampft man das Filtrat ein und chromatographiert den Rückstand mit Hexan/Essigester 9:1 über eine kleine Kieselgelsäule. Nach Eindampfen der Hauptfraktionen erhält man 4,7 g (73,4 %) der Titelverbindung (Verbindung No. 1.113) der Formel

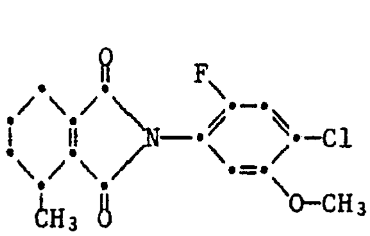

als farbloses klares Oel mit einem $n_D^{21}$ = 1,412.

Analog zu Beispiel 1a und 1b sind die nachstehend genannten Verbindungen der Formel I

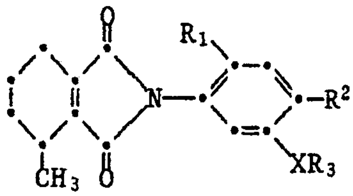 (I) ,

erhältlich.

| Verb.No. | $R_1$ | $R_2$ | X | $R_3$ | phys. Daten |
|---|---|---|---|---|---|
| 1.001 | H | Cl | O | $CH_3$ | |
| 1.002 | H | Cl | O | $C_2H_5$ | |
| 1.003 | H | Cl | O | $n\text{-}C_3H_7$ | |
| 1.004 | H | Cl | O | $i\text{-}C_3H_7$ | |
| 1.005 | H | Cl | O | $n\text{-}C_4H_9$ | |
| 1.006 | H | Cl | O | $i\text{-}C_4H_9$ | |
| 1.007 | H | Cl | O | $s\text{-}C_4H_9$ | |
| 1.008 | H | Cl | O | $t\text{-}C_4H_9$ | |
| 1.009 | H | Cl | O | $n\text{-}C_5H_{11}$ | |
| 1.010 | H | Cl | O | $i\text{-}C_5H_{11}$ | |
| 1.011 | H | Cl | O | Cyclopentyl | |
| 1.012 | H | Cl | O | Cyclohexyl | |
| 1.013 | H | Cl | O | $CH_2\text{-}CH\text{=}CH_2$ | |
| 1.014 | H | Cl | O | $CH_2\text{-}C(CH_3)\text{=}CH_2$ | |
| 1.015 | H | Cl | O | $CH_2\text{-}CH\text{=}CH\text{-}CH_3$ | |
| 1.016 | H | Cl | O | $CH_2\text{-}C\equiv CH$ | |
| 1.017 | H | Cl | O | $CH_2\text{-}C\equiv C\text{-}CH_3$ | |
| 1.018 | H | Br | O | $CH_3$ | |
| 1.019 | H | Br | O | $C_2H_5$ | |
| 1.020 | H | Br | O | $n\text{-}C_3H_7$ | |
| 1.021 | H | Br | O | $i\text{-}C_3H_7$ | |
| 1.022 | H | Br | O | $n\text{-}C_4H_9$ | |
| 1.023 | H | Br | O | $i\text{-}C_4H_9$ | |
| 1.024 | H | Br | O | $n\text{-}C_6H_{13}$ | |
| 1.025 | H | Br | O | $CH_2\text{-}CH\text{=}CH_2$ | |
| 1.026 | H | Br | O | $CH_2\text{-}CH\text{=}CH\text{-}CH_3$ | |
| 1.027 | H | Br | O | $CH_2\text{-}C\equiv CH$ | |

| Verb.No. | $R_1$ | $R_2$ | X | $R_3$ | phys. Daten |
|---|---|---|---|---|---|
| 1.028 | F | Cl | O | $C_2H_5$ | Fp.= 98-100°C |
| 1.029 | F | Cl | O | $n-C_3H_7$ | |
| 1.030 | F | Cl | O | $i-C_3H_7$ | $n_D^{22}$ = 1,5458 |
| 1.031 | F | Cl | O | $n-C_4H_9$ | Fp.= 56-58°C |
| 1.032 | F | Cl | O | $i-C_4H_9$ | Fp.= 79-80°C |
| 1.033 | F | Cl | O | $s-C_4H_9$ | |
| 1.034 | F | Cl | O | $t-C_4H_9$ | |
| 1.035 | F | Cl | O | $n-C_5H_{11}$ | |
| 1.036 | F | Cl | O | $i-C_5H_{11}$ | |
| 1.037 | F | Cl | O | Cyclopentyl | |
| 1.038 | F | Cl | O | Cyclohexyl | |
| 1.039 | F | Cl | O | $n-C_6H_{13}$ | |
| 1.040 | F | Cl | O | $CH_2-CH=CH_2$ | |
| 1.041 | F | Cl | O | $CH_2-C(CH_3)=CH_2$ | |
| 1.042 | F | Cl | O | $CH_2-CH=CH-CH_3$ | |
| 1.043 | F | Cl | O | $CH_2-C\equiv CH$ | |
| 1.044 | F | Cl | O | $CH_2-C\equiv C-CH_3$ | |
| 1.045 | F | Cl | O | 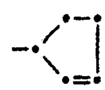 | |
| 1.046 | F | Cl | O | 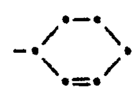 | |
| 1.047 | F | Cl | O | $CH_2COOC_2H_5$ | $n_D^{21}$ = 1,5602 |
| 1.048 | F | Br | O | $CH_3$ | |
| 1.049 | F | Br | O | $C_2H_5$ | |
| 1.050 | F | Br | O | $n-C_3H_7$ | |
| 1.051 | F | Br | O | $i-C_3H_7$ | |
| 1.052 | F | Br | O | $n-C_4H_9$ | |
| 1.053 | F | Br | O | $i-C_4H_9$ | |
| 1.054 | F | Br | O | $s-C_4H_9$ | |
| 1.055 | F | Br | O | $t-C_4H_9$ | |
| 1.056 | F | Br | O | $n-C_5H_{11}$ | |
| 1.057 | F | Br | O | $i-C_5H_{11}$ | |

| Verb.No. | $R_1$ | $R_2$ | X | $R_3$ | phys. Daten |
|---|---|---|---|---|---|
| 1.058 | F | Br | O | Cyclopentyl | |
| 1.059 | F | Br | O | Cyclohexyl | |
| 1.060 | F | Br | O | $n-C_6H_{13}$ | |
| 1.061 | F | Br | O | $CH_2-CH=CH_2$ | |
| 1.062 | F | Br | O | $CH_2-\underset{CH_3}{C}=CH_2$ | |
| 1.063 | F | Br | O | $CH_2-CH=CH-CH_3$ | |
| 1.064 | F | Br | O | $CH_2-C\equiv CH$ | |
| 1.065 | F | Br | O | $CH_2-C\equiv C-CH_3$ | |
| 1.066 | F | Br | O | 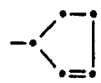 | |
| 1.067 | F | Br | O | 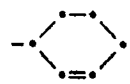 | |
| 1.068 | F | Br | O | $CH_2COOC_2H_5$ | |
| 1.069 | H | Cl | S | $CH_3$ | |
| 1.070 | H | Cl | S | $C_2H_5$ | |
| 1.071 | H | Cl | S | $n-C_3H_7$ | |
| 1.072 | H | Cl | S | $i-C_3H_7$ | |
| 1.073 | H | Cl | S | $n-C_4H_9$ | |
| 1.074 | H | Cl | S | $CH_2-CH=CH_2$ | |
| 1.075 | H | Cl | S | $CH_2-C\equiv CH$ | |
| 1.076 | H | Br | S | $CH_3$ | |
| 1.077 | H | Br | S | $C_2H_5$ | |
| 1.078 | H | Br | S | $n-C_3H_7$ | |
| 1.079 | H | Br | S | $i-C_3H_7$ | |
| 1.080 | H | Br | S | $n-C_4H_9$ | |
| 1.081 | H | Br | S | $CH_2-CH=CH_2$ | |
| 1.082 | H | Br | S | $CH_2-C\equiv CH$ | |
| 1.083 | F | Cl | S | $CH_3$ | |
| 1.084 | F | Cl | S | $C_2H_5$ | |
| 1.085 | F | Cl | S | $n-C_3H_7$ | |
| 1.086 | F | Cl | S | $i-C_3H_7$ | |
| 1.087 | F | Cl | S | $n-C_4H_9$ | |
| 1.088 | F | Cl | S | $i-C_4H_9$ | |
| 1.089 | F | Cl | S | $s-C_4H_9$ | |

| Verb.No. | $R_1$ | $R_2$ | X | $R_3$ | phys. Daten |
|---|---|---|---|---|---|
| 1.090 | F | Cl | S | t-$C_4H_9$ | |
| 1.091 | F | Cl | S | n-$C_5H_{11}$ | |
| 1.092 | F | Cl | S | Cyclopentyl | |
| 1.093 | F | Cl | S | Cyclohexyl | |
| 1.094 | F | Cl | S | $CH_2$-CH=$CH_2$ | |
| 1.095 | F | Cl | S | $CH_2$-C(CH$_3$)=$CH_2$ | |
| 1.096 | F | Cl | S | $CH_2$-CH=CH-$CH_3$ | |
| 1.097 | F | Cl | S | $CH_2$-C≡CH | |
| 1.098 | F | Br | S | $CH_3$ | |
| 1.099 | F | Br | S | $C_2H_5$ | |
| 1.100 | F | Br | S | n-$C_3H_7$ | |
| 1.101 | F | Br | S | i-$C_3H_7$ | |
| 1.102 | F | Br | S | n-$C_4H_9$ | |
| 1.103 | F | Br | S | i-$C_4H_9$ | |
| 1.104 | F | Br | S | s-$C_4H_9$ | |
| 1.105 | F | Br | S | t-$C_4H_9$ | |
| 1.106 | F | Br | S | n-$C_5H_{11}$ | |
| 1.107 | F | Br | S | Cyclopentyl | |
| 1.108 | F | Br | S | Cyclohexyl | |
| 1.109 | F | Br | S | $CH_2$-CH=$CH_2$ | |
| 1.110 | F | Br | S | $CH_2$-C(CH$_3$)=$CH_2$ | |
| 1.111 | F | Br | S | $CH_2$-CH=CH-$CH_3$ | |
| 1.112 | F | Br | S | $CH_2$-C≡CH | |
| 1.113 | F | Cl | O | $CH_3$ | $n_D^{21}$ = 1,412 |

Beispiel 2:
Formulierungsbeispiele

Beispiel 2.1: Formulierungsbeispiele für Wirkstoffe der Formel I (% = Gewichtsprozent)

| a) Emulsionskonzentrate | a) | b) | c) |
|---|---|---|---|
| Wirkstoff gemäss Herstellungsbeispiel 1 | 20 % | 40 % | 50 % |
| Ca-Dodecylbenzolsulfonat | 5 % | 8 % | 5,8 % |

| | | | |
|---|---|---|---|
| Ricinusöl-polyethylenglykolether (36 Mol EO) | 5 % | - | - |
| Tributylphenoyl-polyethylenglykolether (30 mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Herstellungsbeispiel 1 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Herstellungsbeispiel 1 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Herstellungsbeispiel 1 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Herstellungsbeispiel 1 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Herstellungsbeispiel 1 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Herstellungsbeispiel 1 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3:
Biologische Beispiele

Beispiel 3.1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet.

In diesem Versuch zeigen die Verbindungen nach Herstellungsbeispiel 1 starke Herbizidwirkung.

Beispiel 3.2: Post-emergente Herbizid-Wirkung (Kontaktherbizide)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26° C und 45-60 % relativer Luftfeuchtigkeit gehalten. 15 Tage nach der Behandlung wird der Versuch ausgewertet. Auch in diesem Versuch zeigen die Verbindungen nach Herstellungsbeispiel 1 starke bis sehr starke Herbizidwirkung.

Beispiel 3.3: Selektivität gegenüber Getreide im Vorauflauf

In einer Versuchsandordung gemäss Beispiel 3.1 werden Verbindung A (gemäss Beispiel 1.031) und die aus der EP-A 61741 bekannte Verbindung B der Formeln

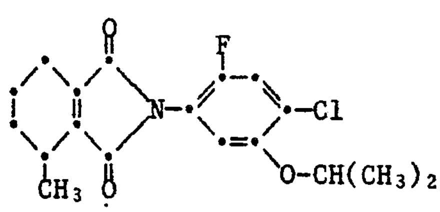

A (Verb. gemäss Beispiel 1.031)

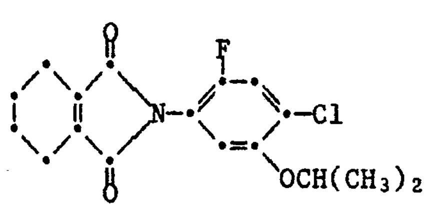

B

bei 1000, 500 und 250 g/ha Aufwandmenge miteinander verglichen.

Der Zustand der Pflanzen wird dabei gemäss folgender Bewertungsskala bonitiert:

1 Pflanze abgestorben oder hat nicht gekeimt

2-8 abnehmende Schadstufen
9 keine Schädigung, die Pflanze gedeiht wie unbehandelte Kontrollpflanzen
Man findet die nachstehend angegebenen Herbizidwirkungen
(1. Spalte 1000 g/ha, 2. Spalte 500 g/ha, 3. Spalte 250 g/ha) für A und B:

| Verbindung | A | | | B | | |
|---|---|---|---|---|---|---|
| | 1. | 2. | 3. Spalte | 1. | 2. | 3. Spalte |
| Weizen | 9 | 9 | 9 | 7 | 7 | 8 |
| Gerste | 9 | 9 | 9 | 2 | 6 | 7 |
| Sinapis | 1 | 1 | 1 | 1 | 1 | 1 |
| Capsella | 1 | 1 | 1 | 1 | 1 | 1 |
| Chenopodium album | 1 | 1 | 1 | 1 | 1 | 1 |
| Chrysanthenum | 1 | 1 | 2 | 1 | 1 | 1 |
| Papaver | 1 | 1 | 1 | 1 | 1 | 1 |
| Poa annua | 1 | 1 | 1 | 1 | 1 | 1 |
| Polygonum | 1 | 1 | 1 | 1 | 1 | 1 |
| Rumex | 1 | 1 | 1 | 1 | 1 | 1 |
| Veronica hedrifolia | 1 | 1 | 1 | 1 | 1 | 6 |
| Veronica persica | 1 | 1 | 1 | 1 | 1 | 1 |
| Viola tricolor | 1 | 1 | 1 | 1 | 1 | 1 |

Beispiel 3.4 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 0,5 bis 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Herstellungsbeispiel 1 schädigen dabei die Unkräuter, nicht aber den Reis.

Beispiel 3.5: Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen gemäss Herstellungsbeispiel 1 in Aufwandmengen bis zu 3000 g/ha behandelten Pflanzen eine deutliche Reduktion des Neuzuwachses, ohne dass dabei die Versuchspflanzen geschädigt wurden.

Beispiel 3.6: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten

Kontrollpflanzen bewirken erfindungsgemässe Wirkstoffe gemäss Herstellungsbeispiel 1 eine merkliche Erhöhung der Anzahl und des Gewichts der Schoten im Haupttrieb.

Beispiel 3.7: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes gemäss Herstellungsbeispiel 1 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Strengeldurchmesser auf.

Beispiel 3.8: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes gemäss Herstellungsbeispiel 1 besprüht. Die Wirkstoffmenge beträgt umgerechnet bis zu 500 g Aktivsubstanz pro Hektar. 21 Tage nach Applikation wird das Wachstum der Gräser beurteilt.

Verbindungen gemäss Herstellungsbeispiel 1 bewirken eine Reduzierung des Neuzuwachses im Vergleich zur unbehandelten Kontrolle.

**Patentansprüche**
**Patentansprüche für folgende Vertragsstaaten : BE, CH, DE, FR, GB, GR, IT, LI, LU, NL, SE**

**1.** Verbindungen der Formel I

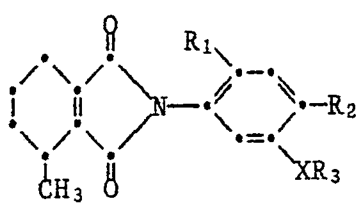

(I)

worin
$R_1$ Wasserstoff; oder Fluor;
$R_2$ Halogen;
X O; oder S;
$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;
bedeutet.

**2.** Verbindung der Formel I nach Anspruch 1, worin
$R_1$ Wasserstoff; oder Fluor;
$R_2$ Fluor; Chlor; oder Brom;
X O; oder S;
$R_3$ $C_3$-$C_5$-Alkenyl; $C_3$-$C_5$-Alkinyl; Cyclopentenyl; Cyclohexenyl; $C_5$-$C_6$-Cycloalkyl; $C_1$-$C_6$-Alkyl welches gewünschtenfalls einfach substituiert ist durch, $C_1$,-$C_4$-Alkoxycarbonyl,
bedeutet.

**3.** Verbindungen der Formel I nach Anspruch 1 oder 2, worin
$R_1$ Fluor
$R_2$ Chlor
$R_3$ Methyl, iso-Butyl, n-Butyl, Isopropyl oder Ethyl

bedeutet.

4. N-(4-Chlor-2-fluor-5-isopropyloxy-phenyl)-3-methyl-3,4,5,6-tetrahydrophthalsäureimid,
N-(4-Chlor-2-fluor-5-methoxy-phenyl)-3-methyl-3,4,5,6-tetrahydrophthalsäureimid,
N-(4-Chlor-5-ethoxy-2-fluor-phenyl)-3-methyl-3,4,5,6-tetrahydrophthalsäureimid,
N-(5-n-Butyl-4-chlor-2-fluor-phenyl)-3-methyl-3,4,5,6-tetrahydrophthalsäureimid,
N-[4-Chlor-4-fluor-5-(2-methylpropyl)-phenyl]-3-methyl-3,4,5,6-tetrahydrophthalsäureimid oder
N-(4-Chlor-5-ethoxycarbonylmethyl-2-fluor-phenyl)-3-methyl-3,4,5,6-tetrahydrophthalsäureimid
als Verbindung der Formel I gemäss Anspruch 1 oder 2.

5. Verfahren zur Herstellung von Verbindungen der Formel I, gemäss Anspruch 1, dadurch gekennezeichnet, das man
a) 3-Methyl-3,4,5,6-tetrahydrophthalsäureanhydrid der Formel II mit einem Anilin der Formel III,

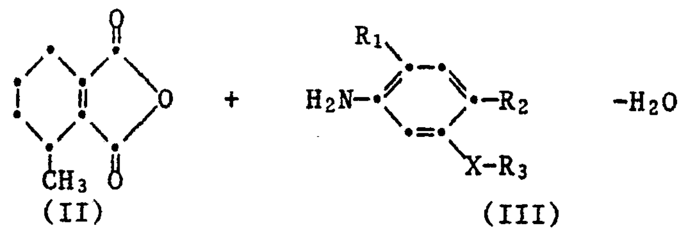


(II) (III) (I)

worin
$R_1$ Wasserstoff oder Fluor,
$R_2$ Halogen,
X O oder S,
$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;
bedeutet, kondensiert oder
b) ein Phenol oder Thiophenol der Formel IV mit einer Verbindung Formel V,

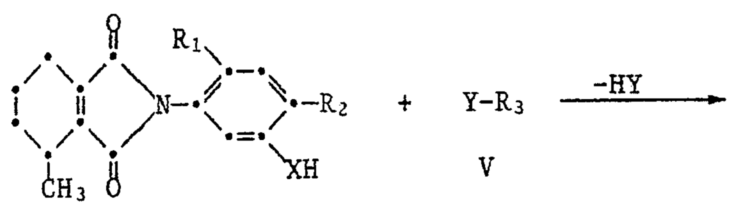


IV V (I)

worin
$R_1$ Wasserstoff oder Fluor,
$R_2$ Halogen,
X O oder S,
$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;
bedeutet, und
Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, umsetzt.

6. Verbindungen der Formel IV

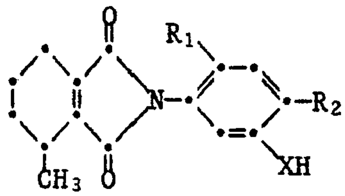

(IV),

worin
R₁ Wasserstoff; oder Fluor;
R₂ Halogen;
X O; oder S;
bedeutet.

7. Verbindungen der Formel IV gemäss Anspruch 6, worin $R_2$ für Fluor, Chlor oder Brom steht.

8. Verfahren zur Herstellung von Verbindungen der Formel IV gemäss Anspruch 6 oder 7, dadurch gekennzeichnet, dass man 3-Methyl-3,4,5,6-tetrahydrophthalsäureanhydrid II mit einem Anilin der Formel VII

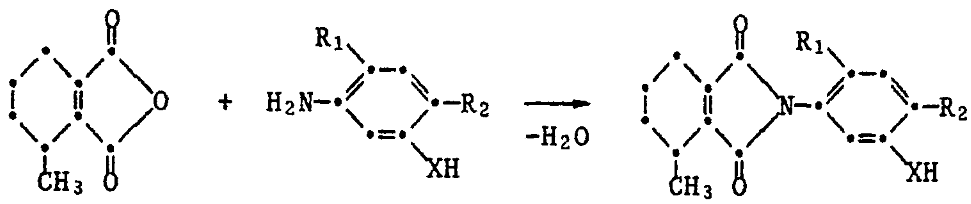


(II) (VII)

worin $R_1$ Wasserstoff oder Fluor, $R_2$ Halogen und X O oder S bedeutet, umsetzt.

9. Ein herbizides oder pflanzenwachstumregulierendes Mittel, dadurch gekennzeichnet, dass es neben Träger- und/oder anderen Hilfsstoffen als Wirkstoff ein N-Phenyltetrahydrophthalimid der Formel I gemäss einem der Ansprüche 1 bis 4 enthält.

10. Die Verwendung eines Wirkstoffes gemäss der Ansprüche 1 bis 4 oder eines einen solchen Wirkstoff enthaltenden Mittels gemäss Anspruch 11 zur Bekämpfung von Unkräutern.

11. Die Verwendung eines Wirkstoffes gemäss einem der Ansprüche 1 bis 4 oder eines einen solchen Wirkstoff enthaltenden Mittels gemäss Anspruch 9 zur Regulierung des Pflanzenwachstums.

12. Ein Verfahren zur Bekämpfung von Unkräutern, dadurch gekennzeichnet, dass man die Unkräuter oder ihren Lebensraum mit einer herbizid wirksamen Menge eines Wirkstoffes der Formel I gemäss einem der Ansprüche 1 bist 4 behandelt.

**Patentansprüche für folgenden Vertragsstaat : AT**

1. Herbizides und/oder pflanzenwuchsregulatorisches Mittel enthaltend eine Verbindung der Formel I

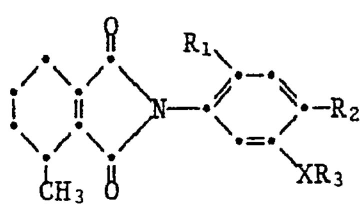

(I)

worin

$R_1$ Wasserstoff; oder Fluor;

$R_2$ Halogen;

X O; oder S;

$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;

bedeutet.

**2.** Mittel nach Anspruch 1, enthaltend eine Verbindung der Formel I, worin

$R_1$ Wasserstoff; oder Fluor;

$R_2$ Fluor; Chlor; oder Brom;

X O; oder S;

$R_3$ $C_3$-$C_5$-Alkenyl; $C_3$-$C_5$-Alkinyl; Cyclopentenyl; Cyclohexenyl; $C_5$-$C_6$-Cycloalkyl; $C_1$-$C_6$-Alkyl welches gewünschtenfalls einfach substituiert ist durch, $C_1$-$C_4$-Alkoxycarbonyl,

bedeutet.

**3.** Mittel nach Anspruch 1 oder 2, enthaltend eine Verbindung der Formel I, worin

$R_1$ Fluor

$R_2$ Chlor

$R_3$ Methyl, iso-Butyl, n-Butyl, Isopropyl oder Ethyl

bedeutet.

**4.** Mittel gemäss Anspruch 1 oder 2, enthaltend

N-(4-Chlor-2-fluor-5-isopropyloxy-phenyl)-3-methyl-3,4,5,6-tetrahydrophthalsäureimid,

N-(4-Chlor-2-fluor-5-methoxy-phenyl)-3-methyl-3,4,5,6-tetrahydrophthalsäureimid,

N-(4-Chlor-5-ethoxy-2-fluor-phenyl)-3-methyl-3,4,5,6-tetrahydrophthalsäureimid,

N-(5-n-Butyl-4-chlor-2-fluor-phenyl)-3-methyl-3,4,5,6-tetrahydrophthalsäureimid,

N-[4-Chlor-4-fluor-5-(2-methylpropyl)-phenyl]-3-methyl-3,4,5,6-tetrahydrophthalsäureimid oder

N-(4-Chlor-5-ethoxycarbonylmethyl-2-fluor-phenyl)-3-methyl-3,4,5,6-tetrahydrophthalsäureimid

als Verbindung der Formel I.

**5.** Verfahren zur Herstellung von Verbindungn der Formel I gemäss Anspruch 1, dadurch gekennzeichnet, dass man

a) 3-Methyl-3,4,5,6-tetrahydrophthalsäureanhydrid der Formel II mit einem Anilin der Formel III,

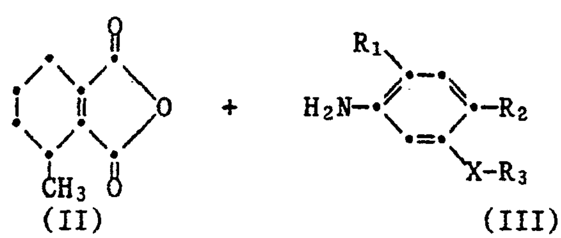

(II) + (III) $-H_2O$ (I)

worin

$R_1$ Wasserstoff oder Fluor,

$R_2$ Halogen

X O oder S,

$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;

bedeutet, kondensiert oder

b) ein Phenol oder Thiophenol der Formel IV mit einer Verbindung der Formel V,

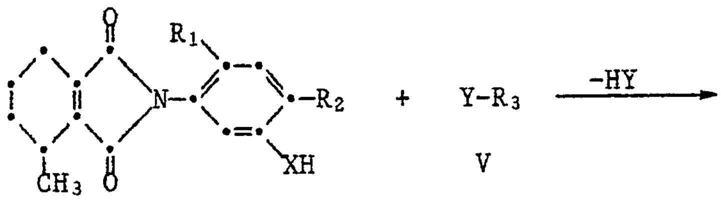

(I)

IV V

worin

$R_1$ Wasserstoff oder Fluor,
$R_2$ Halogen,
X O oder S,
$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;

bedeutet, und

Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, umsetzt.

6. Verfahren zur Herstellung von Verbindungen der Formel IV

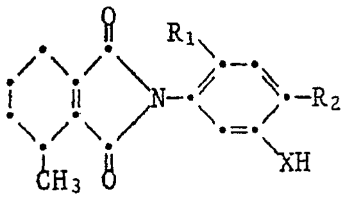
(IV),

worin

$R_1$ Wasserstoff; oder Fluor;
$R_2$ Halogen;
X O; oder S;

bedeutet, dadurch gekennzeichnet, dass man 3-Methyl-3,4,5,6-tetrahydrophthalsäureanhydrid II mit einem Anilin der Formel VII

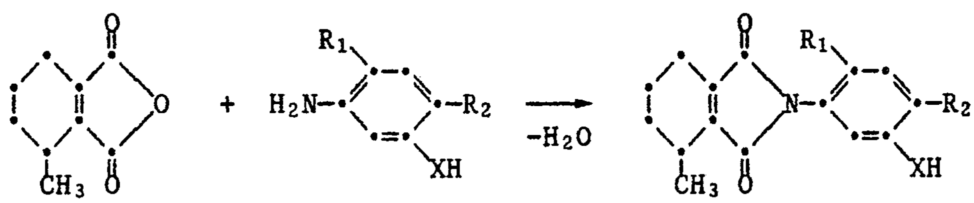

(II) (VII)

worin $R_1$ Wasserstoff oder Fluor, $R_2$ Halogen und X O oder S bedeutet, umsetzt.

7. Verfahren nach Anspruch 6, zur Herstellung von Verbindungen der Formel IV, worin $R_2$ für Fluor, Chlor oder Brom steht.

8. Die Verwendung eines Mittels, gemäss einem der Ansprüche 1 bis 4, zur Bekämpfung von Unkräutern.

9. Die Verwendung eines Mittels, gemäss einem der Ansprüche 1 bis 4, zur Regulierung des Pflanzenwachstums.

10. Ein Verfahren zur Bekämpfung von Unkräutern, dadurch gekennzeichnet, dass man die Unkräuter oder

ihren Lebensraum mit einer herbizid wirksamen Menge eines Wirkstoffes der Formel I gemäss einem der Ansprüche 1 bis 4 behandelt.

**Patentansprüche für folgenden Vertragsstaat : ES**

**1.** Verfahren zur Herstellung von Verbindungen der Formel I.

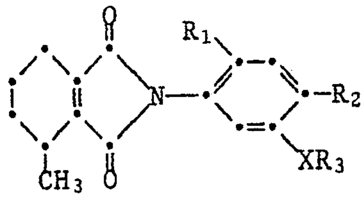

(I)

worin
- $R_1$ Wasserstoff; oder Fluor;
- $R_2$ Halogen;
- X O; oder S;
- $R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;

bedeutet, dadurch gekennzeichnet, dass man

a) 4-Methyl-3,4,5,6-tetrahydrophthalsäureanhydrid der Formel II mit einem Anilin der Formel III,

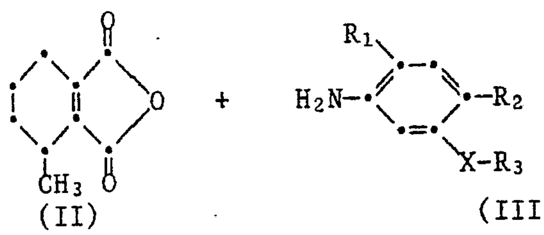

(II) + (III) $-H_2O$ (I)

worin
- $R_1$ Wasserstoff oder Fluor,
- $R_2$ Halogen,
- X O oder S,
- $R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl;

bedeutet, kondensiert oder

b) ein Phenol oder Thiophenol der Formel IV mit einer Verbindung der Formel V,

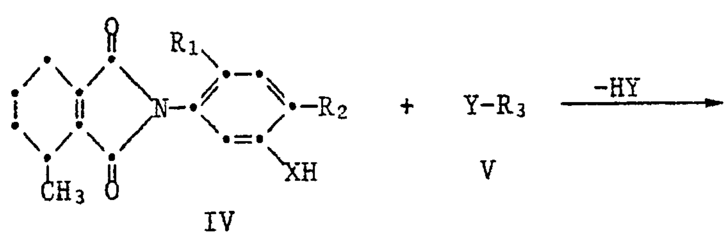


IV + V (I)

worin
- $R_1$ Wasserstoff oder Fluor,
- $R_2$ Halogen,
- X O oder S,

$R_3$ $C_3$-$C_5$-Alkenyl; $C_5$-$C_6$-Cycloalkyl; $C_5$-$C_6$-Cycloalkenyl; $C_3$-$C_5$-Alkinyl; oder $C_1$-$C_6$-Alkyl welches gewünschtenfalls einfach substituiert ist durch, $C_1$-$C_4$-Alkoxycarbonyl,

bedeutet, und

Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, umsetzt.

**2.** Verfahren gemäss Anspruch 1, zur Herstellung von Verbindungen der Formel I, worin

$R_1$ Wasserstoff; oder Fluor;

$R_2$ Fluor; Chlor; oder Brom;

X O; oder S;

$R_3$ $C_3$-$C_5$-Alkenyl; $C_3$-$C_5$-Alkinyl; Cyclopentenyl; Cyclohexenyl; $C_5$-$C_6$-Cycloalkyl; $C_1$-$C_6$-Alkyl welches gewünschtenfalls einfach substituiert ist durch, $C_1$-$C_4$-Alkoxycarbonyl,

bedeutet.

**3.** Verfahren gemäss Anspruch 1 oder 2, zur Herstellung von Verbindungen der Formel I, worin

$R_1$ Fluor

$R_2$ Chlor

$R_3$ Methyl, iso-Butyl, n-Butyl, Isopropyl oder Ethyl

bedeutet.

**4.** Verfahren nach Anspruch 1 oder 2, zur Herstellung von
N(4-Chlor-2-fluor-5-isopropyloxy-phenyl)-3-methyl-3,4,5,6-tetrahydrophthalsäureimid,
N(4-Chlor-2-fluor-5-methoxy-phenyl)-3-methyl-3,4,5,6-tetrahydrophthalsäureimid,
N(4-Chlor-5-ethoxy-2-fluor-phenyl)-3-methyl-3,4,5,6-tetrahydrophthalsäureimid,
N-(5-n-Buty-4-chlor-2-fluor-phenyl)-3-methyl-3,4,5,6-tetrahydrophthalsäureimid,
N-[4-Chlor-4-fluor-5-(2-methylpropyl)-phenyl]-3-methyl-3,4,5,6-tetrahydrophthalsäureimid oder
N-(4-Chlor-5-ethoxycarbonylmethyl-2-fluor-phenyl)-3-methyl-3,4,5,6-tetrahydrophthalsäureimid.

**5.** Verfahren zur Herstellung von Verbindungen der Formel IV

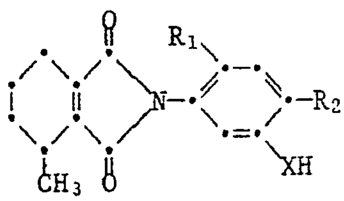

(IV),

worin

$R_1$ Wasserstoff; oder Fluor;

$R_2$ Halogen;

X O; oder S;

bedeutet, dadurch gekennzeichnet, dass man

3-Methyl-3,4,5,6-tetrahydrophthalsäureanhydrid II mit einem Anilin der Formel VII

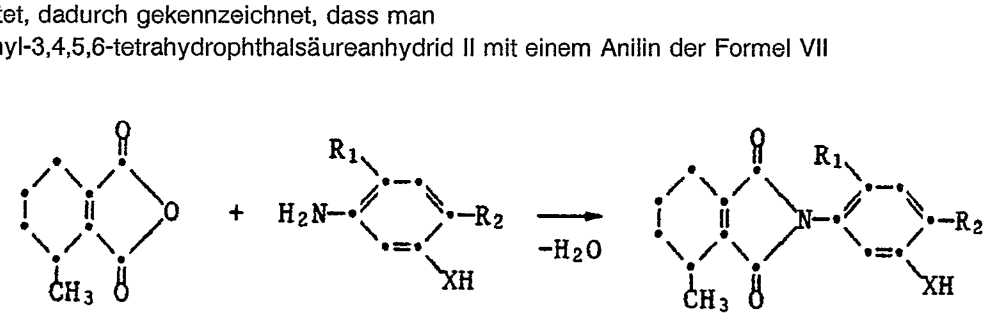


(II) (VII)

worin $R_1$ Wasserstoff oder Fluor, $R_2$ Halogen und X O oder S bedeutet, umsetzt.

6. Ein Verfahren zur Bekämpfung von Unkräutern, dadurch gekennzeichnet, dass man die Unkräuter oder ihren Lebensraum mit einer herbizid wirksamen Menge eines Wirkstoffes der Formel I gemäss einem der Ansprüche 1 bis 4 behandelt.

7. Ein Verfahren zur Regulierung des Pflanzenwachstums, dadurch gekennzeichnet, dass man die Pflanze oder deren Lebensraum mit einer pflanzenwuchsregulatorischen Menge eines Wirkstoffes der Formel I gemäss einem der Ansprüche 1 bis 4 behandelt.

## Claims

## Claims for the following Contracting States : BE, CH, DE, FR, GB, GR, IT, LI, LU, NL, SE

1. A compound of the formula I

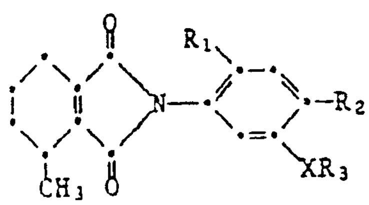

(I)

in which $R_1$ represents hydrogen; or fluorine; $R_2$ representshalogen; X represents O; or S; $R_3$ represents $C_3$-$C_5$-alkenyl; $C_5$-$C_6$cycloalkyl; $C_5$-$C_6$cycloalkenyl; $C_3$-$C_5$-alkynyl; or $C_1$-$C_6$alkyl which, if desired, is monosubstituted by $C_1$-$C_4$alkoxycarbonyl.

2. A compound of the formula I according to claim 1 in which $R_1$ represents hydrogen; or fluorine; $R_2$ represents fluorine; chlorine; or bromine; X represents O; or S; $R_3$ represents $C_3$-$C_5$alkenyl; $C_3$-$C_5$alkynyl; cyclopentenyl; cyclohexenyl; $C_5$-$C_6$cycloalkyl; or $C_1$-$C_6$alkyl which, if desired, is monosubstituted by $C_1$-$C_4$alkoxycarbonyl.

3. A compound of the formula I according to claim 1 or 2 in which $R_1$ represents fluorine, $R_2$ represents chlorine, and $R_3$ represents methyl, isobutyl, n-butyl, isopropyl or ethyl.

4. N-(4-Chloro-2-fluoro-5-isopropoxyphenyl)-3-methyl-3,4,5,6-tetrahydrophthalic acid imide, N-(4-chloro-2-fluoro-5-methoxyphenyl) -3-methyl-3,4,5,6-tetrahydrophthalic acid imide, N-(4-chloro-5-ethoxy-2-fluorophenyl)-3-methyl-3,4,5,6-tetrahydrophthalic acid imide, N-(5-n-butyl-4-chloro-2-fluorophenyl)-3-methyl-3,4,5,6-tetrahydrophthalic acid imide, N-[4-chloro-4-fluoro-5-(2-methylpropyl)-phenyl]-3-methyl-3,4,5,6-tetrahydrophthalic acid imide or N-(4-chloro-5-ethoxycarbonylmethyl-2-fluorophenyl)-3-methyl-3,4,5,6-tetrahydrophthalic acid imide as a compound of the formula I according to claim 1 or 2.

5. A process for the preparation of a compound of the formula I according to claim 1, which comprises
a) condensing 3-methyl-3,4,5,6-tetrahydrophthalic acid anhydride of the formula II with an aniline of the formula III,

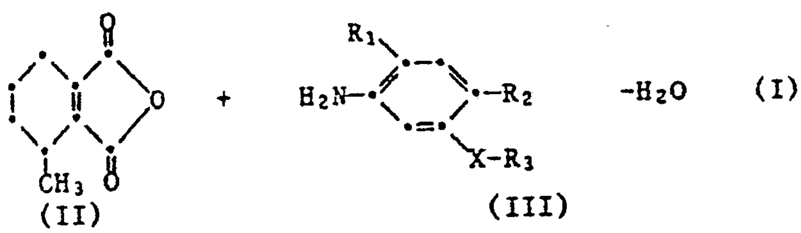


(II) (III)

in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; or
b) reacting a phenol or thiophenol of the formula IV with a compound of the formula V,

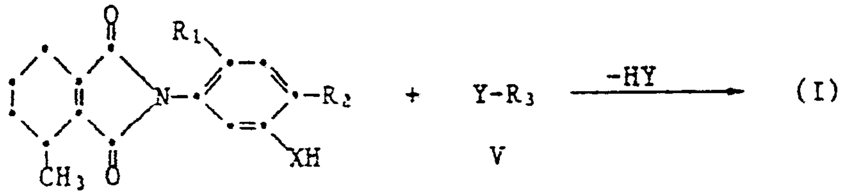


IV V (I)

in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; and Y represents halogen, preferably chlorine, bromine or iodine, or represents a phenylsulphonyl radical which, if desired, is alkylated or halogenated in the phenyl nucleus, or represents a radical of the formula $R_3OSO_2$-O.

**6.** A compound of the formula IV

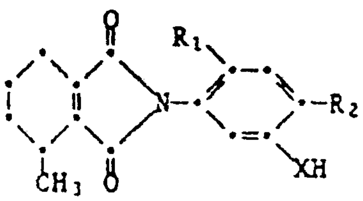

(IV)

in which $R_1$ represents hydrogen; or fluorine; $R_2$ representshalogen; and X represents O; or S.

**7.** A compound of the formula IV according to claim 6 in which $R_2$ represents fluorine, chlorine or bromine.

**8.** A process for the preparation of a compound of the formula IV according to claim 6 or 7, which comprises the reaction of 3-methyl-3,4,5,6-tetrahydrophthalic acid anhydride II with an aniline of the formula VII

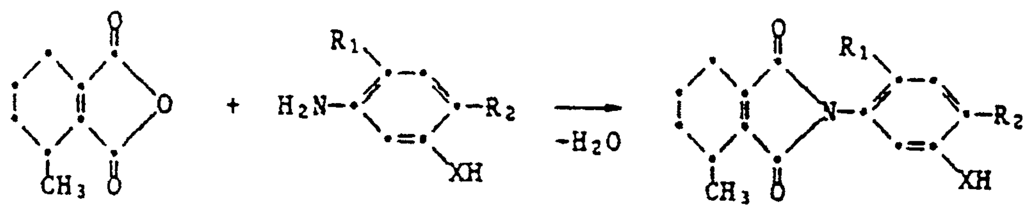


(II) (VII)

in which $R_1$ represents hydrogen or fluorine, $R_2$ represents halogen, and X represents O or S.

**9.** A herbicidal or plant growth regulating composition, which, in addition to carriers and/or other adjuvants, comprises as active ingredient an N-phenyltetrahydrophthalimide of the formula I according to any one of claims 1 to 4.

**10.** The use of an active ingredient according to any one of claims 1 to 4 or of a composition according to claim 11 comprising such an active ingredient for the control of weeds.

**11.** The use of an active ingredient according to any one of claims 1 to 4 or of a composition according to claim 9 comprising such an active ingredient for regulating the growth of plants.

**12.** A method of controlling weeds, which comprises treating the weeds or their growing area with a

herbicidally active amount of an active ingredient of the formula I according to any one of claims 1 to 4.

**Claims for the following Contracting State : AT**

1. A herbicidal and/or plant growth regulating composition comprising a compound of the formula I

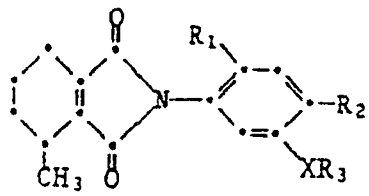

(I)

in which $R_1$ represents hydrogen; or fluorine; $R_2$ representshalogen; X represents O; or S; $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl.

2. A composition according to claim 1, comprising a compound of the formula I in which $R_1$ represents hydrogen; or fluorine; $R_2$ represents fluorine; chlorine; or bromine; X represents O; or S; $R_3$ represents $C_3$-$C_5$alkenyl; $C_3$-$C_5$alkynyl; cyclopentenyl; cyclohexenyl; $C_5$-$C_6$cycloalkyl; or $C_1$-$C_6$alkyl which, if desired, is monosubstituted by $C_1$-$C_4$alkoxycarbonyl.

3. A composition according to claim 1 or 2, comprising a compound of the formula I in which $R_1$ represents fluorine, $R_2$ represents chlorine, and $R_3$ represents methyl, isobutyl, n-butyl, isopropyl or ethyl.

4. A composition according to claim 1 or 2, comprising N-(4-chloro-2-fluoro-5-isopropoxyphenyl)-3-methyl-3,4,5,6-tetrahydrophthalic acid imide, N-(4-chloro-2-fluoro-5-methoxyphenyl)-3-methyl-3,4,5,6-tetrahydrophthalic acid imide, N-(4-chloro-5-ethoxy-2-fluorophenyl)-3-methyl-3,4,5,6-tetrahydrophthalic acid imide, N-(5-n-butyl-4-chloro-2-fluorophenyl)-3-methyl-3, 4,5, 6-tetrahydrophthalic acid imide, N-[4-chloro-4-fluoro-5-(2-methylpropyl)-phenyl]-3-methyl-3,4,5,6-tetrahydrophthalic acid imide or N-(4-chloro-5-ethoxycarbonylmethyl-2-fluorophenyl)-3-methyl-3,4,5,6-tetrahydrophthalic acid imide as a compound of the formula I.

5. A process for the preparation of a compound of the formula I according to claim 1, which comprises
   a) condensing 3-methyl-3,4,5,6-tetrahydrophthalic acid anhydride of the formula II with an aniline of the formula III,

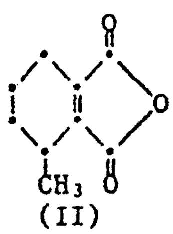

(II)

+

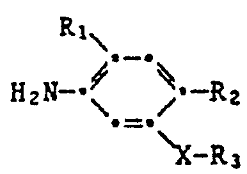

(III)

$-H_2O$ (I)

in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; or
   b) reacting a phenol or thiophenol of the formula IV with a compound of the formula V,

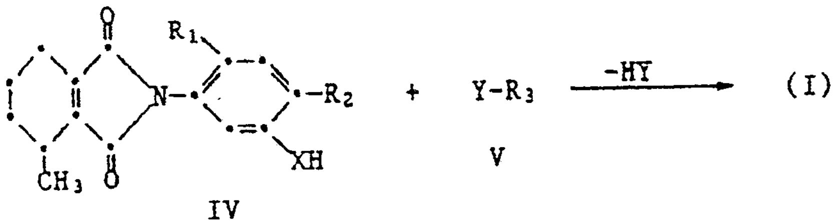


IV + V → (I)

in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; and Y represents halogen, preferably chlorine, bromine or iodine, or represents a phenylsulphonyl radical which, if desired, is alkylated or halogenated in the phenyl nucleus, or represents a radical of the formula $R_3OSO_2$-O.

6. A process for the preparation of a compound of the formula IV

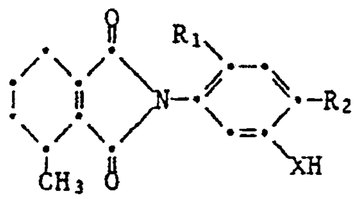

(IV)

in which $R_1$ represents hydrogen; or fluorine; $R_2$ representshalogen; and X represents O; or S; which comprises the reaction of 3-methyl-3,4,5,6-tetrahydrophthalic acid anhydride II with an aniline of the formula VII

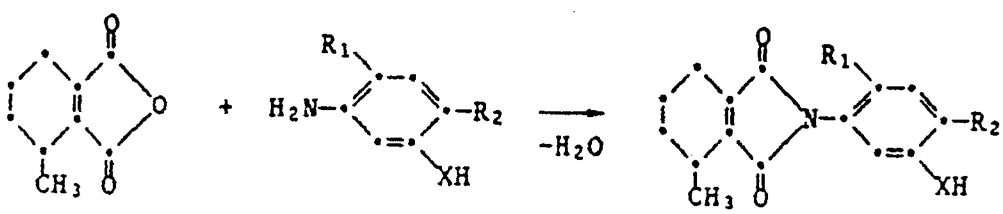


(II) (VII)

in which $R_1$ represents hydrogen or fluorine, $R_2$ represents halogen, and X represents O or S.

7. A process according to claim 6 for the preparation of a compound of the formula IV in which $R_2$ represents fluorine, chlorine or bromine.

8. The use of a composition according to any one of claims 1 to 4 for the control of weeds.

9. The use of a composition according to any one of claims 1 to 4 for regulating the growth of plants.

10. A method of controlling weeds, which comprises treating the weeds or their growing area with a herbicidally active amount of an active ingredient of the formula I according to any one of claims 1 to 4.

**Claims for the following Contracting State : ES**

1. A process for the preparation of a compound of the formula I

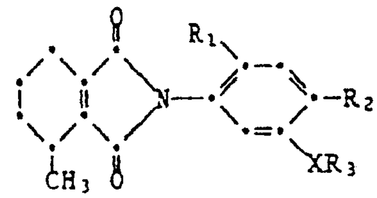

(I)

in which $R_1$ represents hydrogen; or fluorine; $R_2$ representshalogen; X represents O; or S; $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl, which comprises

a) condensing 4-methyl-3,4,5,6-tetrahydrophthalic acid anhydride of the formula II with an aniline of the formula III,

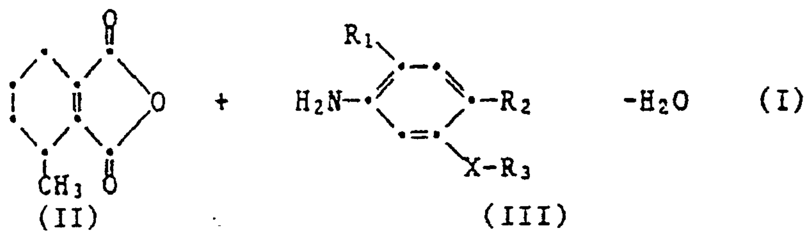


in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; or

b) reacting a phenol or thiophenol of the formula IV with a compound of the formula V,

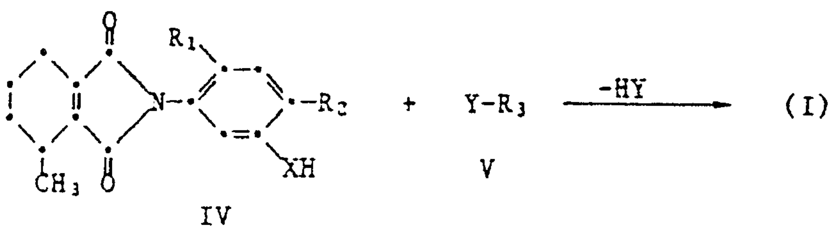


in which $R_1$ represents hydrogen or fluorine, $R_2$ represents halogen, X represents O or S, $R_3$ represents $C_3$-$C_5$alkenyl; $C_5$-$C_6$cycloalkyl; $C_5$-$C_6$cycloalkenyl; $C_3$-$C_5$alkynyl; or $C_1$-$C_6$alkyl which, if desired, is monosubstituted by $C_1$-$C_4$alkoxycarbonyl; and Y represents halogen, preferably chlorine, bromine or iodine, or represents a phenylsulphonyl radical which, if desired, is alkylated or halogenated in the phenyl nucleus, or represents a radical of the formula $R_3OSO_2$-O.

**2.** A process according to claim 1 for the preparation of a compound of the formula I in which $R_1$ represents hydrogen; or fluorine; $R_2$ represents fluorine; chlorine; or bromine; X represents O; or S; $R_3$ represents $C_3$-$C_5$alkenyl; $C_3$-$C_5$alkynyl; cyclopentenyl; cyclohexenyl; $C_5$-$C_6$cycloalkyl; or $C_1$-$C_6$alkyl which, if desired, is monosubstituted by $C_1$-$C_4$alkoxycarbonyl.

**3.** A process according to claim 1 or 2 for the preparation of a compound of the formula I in which $R_1$ represents fluorine, $R_2$ represents chlorine, and $R_3$ represents methyl, isobutyl, n-butyl, isopropyl or ethyl.

**4.** A process according to claim 1 or 2 for the preparation of N-(4-chloro-2-fluoro-5-isopropoxyphenyl)-3-methyl-3,4,5,6-tetrahydrophthalic acid imide, N-(4-chloro-2-fluoro-5-methoxyphenyl)-3-methyl-3,4,5,6-tetrahydrophthalic acid imide, N-(4-chloro-5-ethoxy-2-fluorophenyl)-3-methyl-3,4,5,6-tetrahydrophthaic acid imide, N-(5-n-butyl-4-chloro-2-fluorophenyl)-3-methyl-3,4,5,6-tetrahydrophthalic acid imide, N-[4-chloro-4-fluoro-5-(2-methylpropyl)-phenyl]-3-methyl-3,4,5,6-tetrahydrophthalic acid imide or N-(4-chloro-5-ethoxy-carbonylmethyl-2-fluorophenyl)-3-methyl-3,4,5,6-tetrahydrophthalic acid imide.

**5.** A process for the preparation of a compound of the formula IV

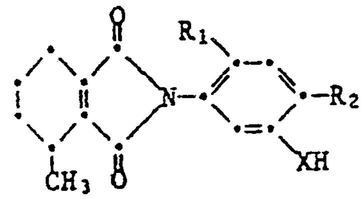

(IV)

in which $R_1$ represents hydrogen; or fluorine; $R_2$ representshalogen; and X represents O; or S; which comprises the reaction of 3-methyl-3,4,5,6-tetrahydrophthalic acid anhydride II with an aniline of the formula VII

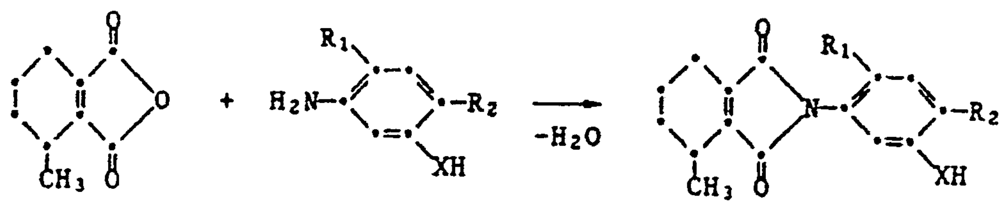


(II) (VII)

in which $R_1$ represents hydrogen or fluorine, $R_2$ represents halogen, and X represents O or S.

**6.** A method of controlling weeds, which comprises treating the weeds or their growing area with a herbicidally active amount of an active ingredient of the formula I according to any one of claims 1 to 4.

**7.** A method of regulating the growth of plants, which comprises treating the plant or its growing area with a plant growth regulating amount of an active ingredient of the formula I according to any one of claims 1 to 4.

**Revendications**

**Revendications pour les Etats contractants suivants : BE, CH, DE, FR, GB, GR, IT, LI, LU, NL, SE**

**1.** Composés de formule I

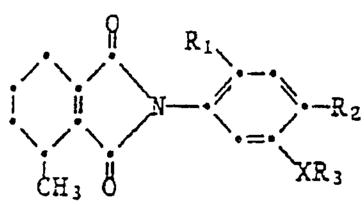

(I)

dans laquelle

- $R_1$ représente l'hydrogène ou le fluor,
- $R_2$ représente un halogène,
- X représente O ou S,
- $R_3$ représente un groupe alcényle en C3-C5; cycloalkyle en C5-C6; cycloalcényle en C5-C6; alcynyle en C3-C5; ou bien un groupe alkyle en C1-C6 portant éventuellement un substituant (alcoxy en C1-C4)-carbonyle.

2. Composé de formule I selon la revendication 1, dans laquelle

$R_1$ représente l'hydrogène ou le fluor,

$R_2$ représente le fluor, le chlore ou le brome,

X représente O ou S,

$R_3$ représente un groupe alcényle en C3-C5; alcynyle en C3-C5; cyclopentényle; cyclohexényle; cycloalkyle en C5-C6; un groupe alkyle en C1-C6 portant éventuellement un substituant (alcoxy en C1-C4)-carbonyle.

3. Composés de formule I selon la revendication 1 ou 2, dans laquelle

$R_1$ représente le fluor,

$R_2$ représente le chlore,

$R_3$ représente un groupe méthyle, isobutyle, n-butyle, isopropyle ou éthyle.

4. Le N-(4-chloro-2-fluoro-5-isopropyloxyphényl)-3-méthyl-3,4,5,6-tétrahydrophtalimide,
le N-(4-chloro-2-fluoro-5-méthoxyphényl)-3-méthyl-3,4,5, 6-tétrahydrophtalimide,
le N-(4-chloro-5-éthoxy-2-fluorophényl)-3-méthyl-3,4,5,6-tétrahydrophtalimide,
le N-(5-n-butyl)4-chloro-2-fluorophényl)-3-méthyl-3,4,5,6-tétrahydrophtalimide,
le N-[4-chloro-4-fluoro-5-(2-méthylpropyl)-phényl]-3-méthyl-3,4,5,6-tétrahydrophtalimide, ou
le N-(4-chloro-5-éthoxycarbonylméthyl-2-fluorophényl)-3-méthyl-3,4,5,6-tétrahydrophtalimide,
en tant que composé de formule I selon la revendication 1 ou 2.

5. Procédé de préparation des composés de formule I de la revendication 1, caractérisé en ce que :

a) on condense l'anhydride 3-méthyl-3,4,5,6-tétrahydrophtalique de formule II avec une aniline de formule III

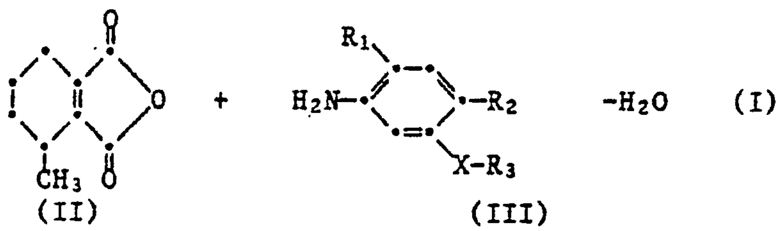


(II) (III) (I)

$R_1$ représentant l'hydrogène ou le fluor,

$R_2$ un halogène,

X représentant O ou S, et

$R_3$ un groupe alcényle en C3-C5; cycloalkyle en C5-C6; cycloalcényle en C5-C6; alcynyle en C3-C5; ou un groupe alkyle en C1-C6 portant éventuellement un substituant (alcoxy en C1-C4)-carbonyle, ou bien

b) on fait réagir un phénol ou thiophénol de formule IV avec un composé de formule V

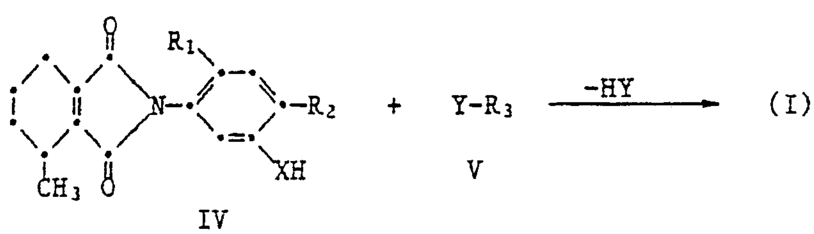


IV V (I)

$R_1$ représente l'hydrogène ou le fluor,

$R_2$ un halogène,

X représentant O ou S,

$R_3$ représentant un groupe alcényle en C3-C5; cycloalkyle en C5-C6; cycloalcényle en C5-C6; alcynyle en C3-C5; ou un groupe alkyle en C1-C6 portant éventuellement un substituant (alcoxy en C1-C4)-carbonyle, et

Y représente un halogène, de préférence le chlore, le brome ou l'iode ou un groupe phénylsulfonyle éventuellement alkylé ou halogéné dans le noyau phényle, ou un radical de formule $R_3OSO_2$-O.

6. Composés de formule IV

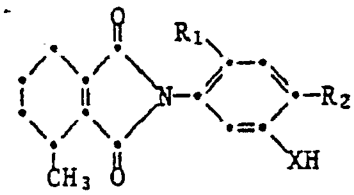

(IV),

dans laquelle

$R_1$ représente l'hydrogène ou le fluor,
$R_2$ représente un halogène,
X représente O ou S.

7. Composés de formule IV de la revendication 6, dans laquelle $R_2$ représente le fluor, le chlore ou le brome.

8. Procédé de préparation des composés de formule IV de la revendication 6 ou 7, caractérisé en ce que l'on fait réagir l'anhydride 3-méthyl-3,4,5,6-tétrahydrophtalique II avec une aniline de formule VII

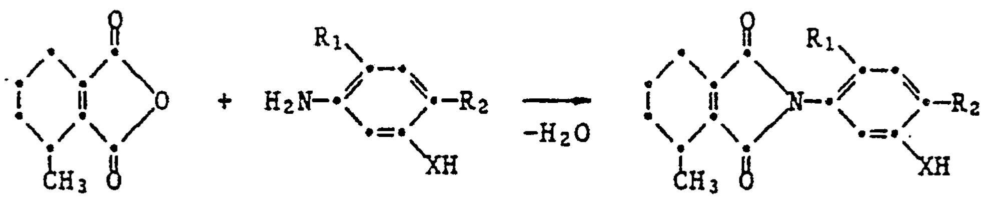


(II) (VII)

$R_1$ représentant l'hydrogène ou le fluor, $R_2$ un halogène et X O ou S.

9. Un produit herbicide ou régulateur de la croissance des végétaux, caractérisé en ce qu'il contient en tant que substance active, avec des véhicules et/ou d'autres produits auxiliaires, un N-phényltétrahydrophtalimide de formule I selon l'une des revendications 1 à 4.

10. L'utilisation d'une substance active selon l'une des revendications 1 à 4 ou d'un produit contenant une telle substance active selon la revendication 11, pour la lutte contre les végétaux adventices.

11. L'utilisation d'une substance active selon l'une des revendications 1 à 4 ou d'un produit contenant une telle substanoe active selon revendication 9, pour la régulation de la croissance des végétaux.

12. Un procédé pour combattre les végétaux adventices, caractérisé en ce que l'on traite les végétaux adventices ou leur habitat par une quantité herbicide efficace d'une substance active de formule I selon une des revendications 1 à 4.

**Revendications pour l'Etat contractant suivant : AT**

1. Produit herbicide et/ou régulateur de la croissance des végétaux contenant un composé de formule I

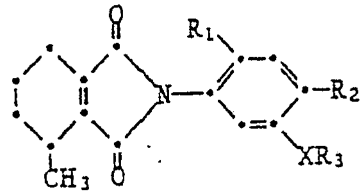

(I)

dans laquelle

$R_1$ représente l'hydrogène ou le fluor,

$R_2$ représente un halogène,

X représente O ou S,

$R_3$ représente un groupe alcényle en C3-C5; cycloalkyle en C5-C6; cycloalcényle en C5-C6; alcynyle en C3-C5; ou bien un groupe alkyle en C1-C6 portant éventuellement un substituant (alcoxy en C1-C4)-carbonyle.

**2.** Produit selon la revendication 1, contenant un composé de formule I dans laquelle

$R_1$ représente l'hydrogène ou le fluor,

$R_2$ représente le fluor, le chlore ou le brome,

X représente O ou S,

$R_3$ représente un groupe alcényle en C3-C5; alcynyle en C3-C5; cyclopentényle; cyclohexényle; cycloalkyle en C5-C6; un groupe alkyle en C1-C6 portant éventuellement un substituant (alcoxy en C1-C4)-carbonyle.

**3.** Produit selon la revendication 1 ou 2, contenant un composé de formule I dans laquelle

$R_1$ représente le fluor,

$R_2$ représente le chlore,

$R_3$ représente un groupe méthyle, isobutyle, n-butyle, isopropyle ou éthyle.

**4.** Produit selon revendication 1 ou 2, contenant
le N-(4-chloro-2-fluoro-5-isopropyloxyphényl)-3-méthyl-3,4,5,6-tétrahydrophtalimide,
le N-(4-chloro-2-fluoro-5-méthoxyphényl)-3-méthyl-3,4, 5,6-tétrahydrophtalimide,
le N-(4-chloro-5-éthoxy-2-fluorophényl)-3-méthyl-3,4,5,6-tétrahydrophtalimide,
le N-(5-n-butyl)-4-chloro-2-fluorophényl)-3-méthyl-3,4,5,6-tétrahydrophtalimide,
le N-[4-chloro-4-fluoro-5-(2-méthylpropyl)-phényl]-3-méthyl-3,4,5,6-tétrahydrophtalimide, ou
le N-(4-chloro-5-éthoxycarbonylméthyl-2-fluorophényl)-3-méthyl-3,4,5,6-tétrahydrophtalimide,
en tant que composé de formule I.

**5.** Procédé de préparation des composés de formule I de la revendication 1, caractérisé en ce que :

a) on condense l'anhydride 3-méthyl-3,4,5,6-tétrahydrophtalique de formule II avec une aniline de formule III

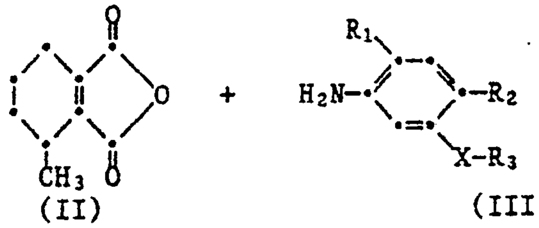

(II) (III)

$-H_2O$ (I)

$R_1$ représentant l'hydrogène ou le fluor,

$R_2$ un halogène,

X représentant O ou S, et

$R_3$ un groupe alcényle en C3-C5; cycloalkyle en C5-C6; cycloalcényle en C5-C6; alcynyle en C3-C5; ou un groupe alkyle en C1-C6 portant éventuellement un substituant (alcoxy en C1-C4)-carbonyle, ou bien

b) on fait réagir un phénol ou thiophénol de formule IV avec un composé de formule V

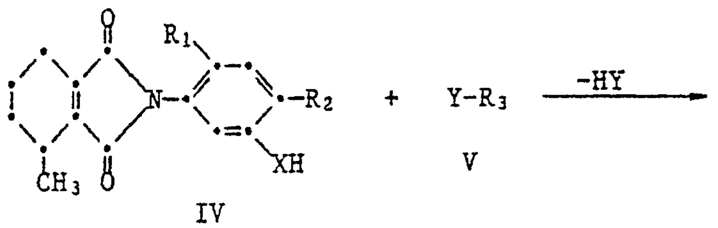


IV V (I)

| | |
|---|---|
| $R_1$ | représente l'hydrogène ou le fluor, |
| $R_2$ | un halogène, |
| X | représentant O ou S, |
| $R_3$ | représentant un groupe alcényle en C3-C5; cycloalkyle en C5-C6; cycloalcényle en C5-C6; alcynyle en C3-C5; ou un groupe alkyle en C1-C6 portant éventuellement un substituant (alcoxy en C1-C4)-carbonyle, et |
| Y | représente un halogène, de préférence le chlore, le brome ou l'iode ou un groupe phénylsulfonyle éventuellement alkylé ou halogéné dans le noyau phényle, ou un radical de formule $R_3OSO_2$-O. |

**6.** Procédé de préparation des composés de formule IV

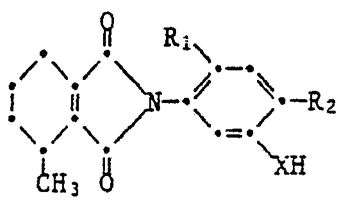

(IV),

dans laquelle

| | |
|---|---|
| $R_1$ | représente l'hydrogène ou le fluor, |
| $R_2$ | représente un halogène, |
| X | représente O ou S, |

caractérisé en ce que l'on fait réagir l'anhydride 3-méthyl-3,4,5,6-tétrahydrophtalique II avec une aniline de formule VII

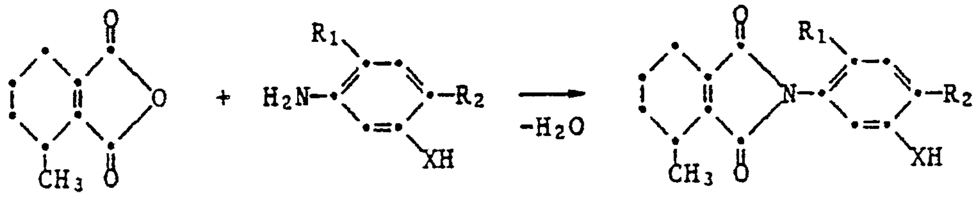


(II) (VII)

$R_1$ représentant l'hydrogène ou le fluor, $R_2$ un halogène et X O ou S.

**7.** Procédé selon la revendication 6, pour la préparation des composés de formule IV dans laquelle $R_2$ représente le fluor, le chlore ou le brome.

**8.** L'utilisation d'un produit selon l'une des revendications 1 à 4 pour la lutte contre les végétaux adventices.

**9.** L'utilisation d'un produit selon l'une des revendications 1 à 4 pour la régulation de la croissance des végétaux.

10. Un procédé pour combattre les végétaux adventices, caractérisé en ce que l'on traite les végétaux adventices ou leur habitat par une quantité herbicide efficace d'une substance active de formule I selon l'une des revendications 1 à 4.

**Revendications pour l'Etat contractant suivant : ES**

1. Procédé de préparation des composés de formule I

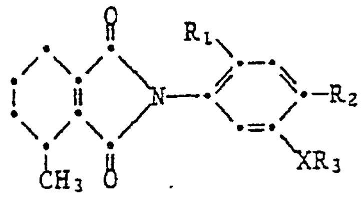

(I)

dans laquelle

- $R_1$ représente l'hydrogène ou le fluor,
- $R_2$ représente un halogène,
- X représente O ou S,
- $R_3$ représente un groupe alcényle en C3-C5; cycloalkyle en C5-C6; cycloalcényle en C5-C6; alcynyle en C3-C5; ou bien un groupe alkyle en C1-C6 portant éventuellement un substituant (alcoxy en C1-C4)-carbonyle.

caractérisé en ce que :

a) on condense l'anhydride 4-méthyl-3,4,5,6-tétrahydrophtalique de formule II avec une aniline de formule III

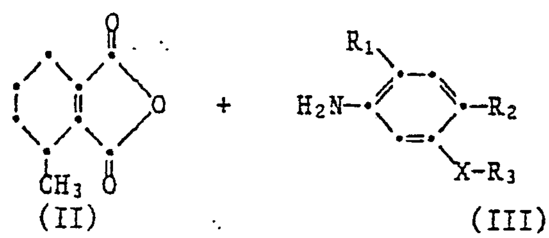

$-H_2O$ (I)

(II) (III)

- $R_1$ représentant l'hydrogène ou le fluor,
- $R_2$ un halogène,
- X représentant O ou S, et
- $R_3$ un groupe alcényle en C3-C5; cycloalkyle en C5-C6; cycloalcényle en C5-C6; alcynyle en C3-C5; ou un groupe alkyle en C1-C6 portant éventuellement un substituant (alcoxy en C1-C4)-carbonyle, ou bien

b) on fait réagir un phénol ou thiophénol de formule IV avec un composé de formule V

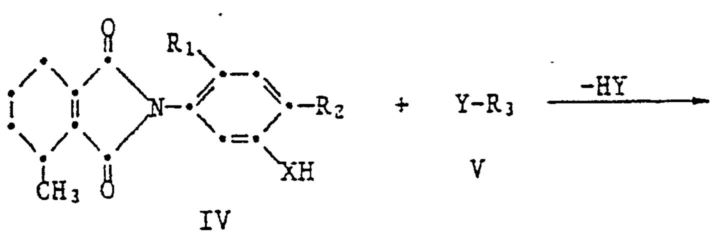


(I)

IV

- $R_1$ représente l'hydrogène ou le fluor,
- $R_2$ un halogène,
- X représentant O ou S,
- $R_3$ représentant un groupe alcényle en C3-C5; cycloalkyle en C5-C6; cycloalcényle en C5-C6;

alcynyle en C3-C5; ou un groupe alkyle en C1-C6 portant éventuellement un substituant (alcoxy en C1-C4)-carbonyle, et

Y représente un halogène, de préférence le chlore, le brome ou l'iode ou un groupe phénylsulfonyle éventuellement alkylé ou halogéné dans le noyau phényle, ou un radical de formule $R_3OSO_2$-O.

**2.** Procédé selon la revendication 1, pour la préparation des composés de formule I dans laquelle

$R_1$ représente l'hydrogène ou le fluor;
$R_2$ représente le fluor, le chlore ou le brome;
X représente O ou S;
$R_3$ représentant un groupe alcényle en C3-C5; alcynyle en C3-C5; cyclopentényle; cyclohexényle; cycloalkyle en C5-C6; alkyle en C1-C6 portant éventuellement un substituant alcoxycarbonyle en C1-C4.

**3.** Procédé selon la revendication 1 ou 2, pour la préparation des composés de formule I dans laquelle

$R_1$ représente le fluor,
$R_2$ représente le chlore,
$R_3$ représente un groupe méthyle, isobutyle, n-butyle, isopropyle ou éthyle.

**4.** Procédé selon la revendication 1 ou 2, pour la préparation de
N-(4-chloro-2-fluoro-5-isopropyloxyphényl)-3-méthyl-3,4,5,6-tétrahydrophtalimide,
N-(4-chloro-2-fluoro-5-méthoxyphényl)-3-méthyl-3,4,5,6-tétrahydrophtalimide,
N-(4-chloro-5-éthoxy-2-fluorophényl)-3-méthyl-3,4,5,6-tétrahydrophtalimide,
N-(5-n-butyl)-4-chloro-2-fluorophényl)-3-méthyl-3,4,5,6-tétrahydrophtalimide,
N-[4-chloro-4-fluoro-5-(2-méthylpropyl)-phényl]-3-méthyl-3,4,5,6-tétrahydrophtalimide, ou
N-(4-chloro-5-éthoxycarbonylméthyl-2-fluorophényl)-3-méthyl-3,4,5,6-tétrahydrophtalimide.

**5.** Procédé de préparation des composés de formule IV

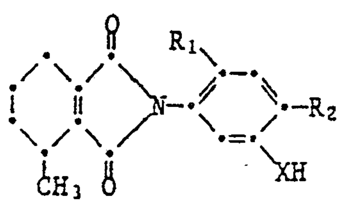

(IV),

dans laquelle

$R_1$ représente l'hydrogène ou le fluor,
$R_2$ représente un halogène,
X représente O ou S,

caractérisé en ce que l'on fait réagir l'anhydride 3-méthyl-3,4,5,6-tétrahydrophtalique II avec une aniline de formule VII

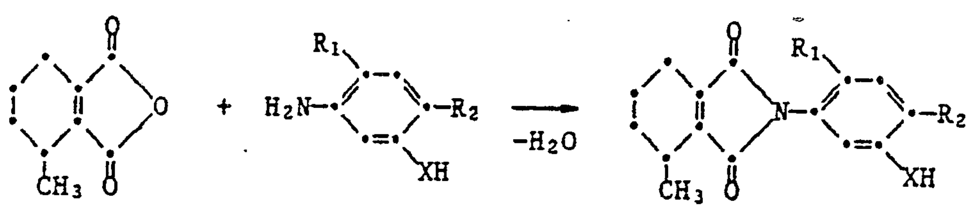


(II) (VII)

$R_1$ représentant l'hydrogène ou le fluor,
$R_2$ un halogène et X O ou S.

**6.** Un procédé pour combattre les végétaux adventices, caractérisé en ce que l'on traite les végétaux

adventices ou leur habitat par une quantité herbicide efficace d'une substance active de formule I selon l'une des revendications 1 à 4.

7. Un procédé pour la régulation de la croissance des végétaux, caractérisé en ce que l'on traite les plantes ou leur habitat par une quantité efficace d'une substance active de formule I selon l'une des revendications 1 à 4.